# EUROPÄISCHE PATENTANMELDUNG

(11) **EP 1 077 374 A2**
(43) Veröffentlichungstag der Anmeldung: **21.02.2001**
(21) Anmeldenummer: 00113616.7
(22) Anmeldetag: 28.06.2000
(51) Int. Cl.: G01N 27/411

(54) **Sensor und Verfahren zur Bestimmung des Magnesiumgehaltes einer Gusseisenschmelze**

(30) Priorität: 18.08.1999 DE 19938580
(71) Anmelder: Georg Fischer Fahrzeugtechnik AG, 8201 Schaffhausen (CH)
(72) Erfinder: Menk, Werner, 8200 Schaffhausen (CH); Gerber, Urs, 8247 Flurlingen (CH); Kleiner, Simon, 8044 Zürich (CH)
(74) Vertreter: Weiss, Wolfgang, Dr.

(57) **Zusammenfassung**

Der prozentuale Anteil des gelösten Magnesiums ist ein kritischer Faktor bei der Herstellung von Gusseisenlegierungen, die eine metallographische Struktur mit Kugelgraphit oder Vermikulargraphit aufweisen. Es wird einen Sensor (1) vorgeschlagen zur Bestimmung des Magnesiumgehaltes einer Gusseisenschmelze, der einen chemisch inerten Träger (2) umfasst, auf dem eine Mischung (3) von Metalloxiden und Schwefel aufbringbar ist, wobei die Mischung (3) mit dem gelösten Magnesium der Gusseisenschmelze in Abhängigkeit des Gehaltes an gelöstem Magnesium unter Farbänderung irreversibel reagiert. Die Auswertung der Farbänderung wird unter Verwendung einer Computersoftware für digitale Farbbildbearbeitung durchgeführt.

## Beschreibung

Die Erfindung bezieht sich auf einen Sensor und ein Verfahren zur Bestimmung des Magnesiumgehaltes einer Gusseisenschmelze.

Es sind Verfahren zur Bestimmung des Magnesiumgehaltes einer Gusseisenschmelze bekannt, die eine Probenahme und eine Analyse der Probe in einem Spektrometer umfassen. Eine Probe, die aus der Schmelze entnommen wird, wird abgekühlt und in einem Emissionsspektrometer auf die Metallanteile analysiert. Eine quantitative Analyse ist möglich durch Vergleich der Intensität der Spektrallinien mit Referenzen bekannter Zusammensetzungen. Die Spektralanalyse unterscheidet nicht zwischen chemisch beispielsweise an Sauerstoff oder Schwefel gebundenem und gelöstem metallischem Magnesium.

Aus der US -A- 5 632 881 sind einen Sensor und ein Verfahren zur Bestimmung des Magnesiumgehaltes einer Aluminiumschmelze bekannt, wobei zwei gegenüber der Schmelze chemisch inerte Elektroden verwendet werden. Eine Elektrode wird in dem geschmolzenen Aluminium eingetaucht und die zweite Elektrode wird in einer geschmolzenen Salzschicht eingetaucht, die das geschmolzene Aluminium überdeckt. Die zweite Elektrode ist von einem Behälter umgeben, der mit einem Referenzmaterial gefüllt ist. Durch die elektrische Verbindung der zwei Elektroden wird ein elektrochemisches Element gebildet, in dem ein elektrischer Strom eine Potentialänderung in Abhängigkeit des Magenesiumgehaltes in der Aluminiumschmelze erzeugt. Der Sensor und das Verfahren werden verwendet um in einem Verfahren zur elektrolytischen Beseitigung von Magnesiumanteilen in Abfallaluminiumschmelzen die Magnesiumgehalte bis hinunter zu 1000 ppm Mg zu bestimmen.

Ausgehend von diesem Stand der Technik ist es Aufgabe der Erfindung, einen Sensor und ein Verfahren zur Bestimmung des Magnesiumgehaltes einer Gusseisenschmelze anzugeben, die einfach und kostengünstig sind und bei möglichst niedrigen Konzentrationen anwendbar sind.

Diese Aufgabe wird gelöst durch einen Sensor zur Bestimmung des Magnesiumgehaltes einer Gusseisenschmelze umfassend ein chemisch inerter Träger, auf dem eine Mischung von Metalloxiden und Schwefel aufbringbar ist, wobei die Mischung mit dem gelösten Magnesium der Gusseisenschmelze in Abhängigkeit des Gehaltes an gelöstem Magnesium unter Farbänderung irreversibel reagiert.

Bezüglich des Verfahrens wird die Aufgabe gelöst durch ein Verfahren zur Bestimmung des Magnesiumgehaltes einer Gusseisenschmelze unter Verwendung des Sensors umfassend folgende Schritte: Eintauchen des Sensors in die Metallschmelze während 2 bis 20 Sekunden, Kühlen des Sensors bis auf Raumtemperatur, Beleuchten des Sensors mit einer Standardlichtquelle, Fotografieren des Sensors mit einem Farbfotovefahren, Auswerten der Farbe und der Helligkeit des Sensors unter Verwendung einer Standardfarbskala und Vergleichen der Farb- und Helligkeitswerte mit einer Vergleichskurve.

Bevorzugte Weiterbildungen der Erfindung ergeben sich aus den abhängigen Ansprüchen.

Es ist von Vorteil, dass die Bestimmung des Magnesiumgehaltes mit allgemein erhältlichen Hilfsmitteln durchgeführt werden kann. Dies wird dadurch erreicht, dass zur Herstellung des Sensors der inerte Träger in eine Lösung oder eine Suspension von Metalloxiden und Schwefel eingetaucht und anschliessend getrocknet wird, derart dass dabei eine Beschichtung des Trägermaterials mit der Mischung erhalten wird. Dies wird auch dadurch erreicht, dass die Verfahrensschritte Fotografieren, Auswerten und Vergleichen unter Verwendung einer digitalen Videokamera und einer damit gekoppelten Computersoftware für digitale Farbbildbearbeitung durchgeführt werden.

Die Herstellung des Sensors ist einfach und die Auswertung ist leicht und reproduzierbar. Die Lösung oder die Suspension von Metalloxiden und Schwefel wird, wie eine Schlichte, in einer in Giessereien bekannten Technik, hergestellt und auf einem handelsüblichen Trägermaterial, beispielsweise ein Schutzrohr für Thermoelemente, das in der Giesserei ohnehin verwendet wird, aufgebracht. Die Auswertung der Farbänderung wird unter Verwendung einer handelsüblichen Computersoftware durchgeführt.

Der erfindungsgemässe Sensor und das Verfahren liefern reproduzierbare Werte für den Magnesiumgehalt einer Gusseisenschmelze auch bei sehr niedrigen Magnesiumkonzentrationen von beispielsweise 50 ppm Mg.

Das erfindungsgemässe Verfahren hat gegenüber bestehenden Verfahren, die eine Probenahme und eine Analyse der Probe in einem Spektrometer umfassen, den für die Prozessführung wichtigen Vorteil, dass lediglich die in der Schmelze gelösten Magnesiumanteile und nicht die Summe der in der Schmelze vorhandenen Magnesiumanteile von gelöstem plus gebundenem Magnesium bestimmt wird. Der prozentuale Anteil des gelösten Magnesiums ist ein kritischer Faktor bei der Herstellung von Gusseisenlegierungen, die eine metallographische Struktur mit Kugelgraphit oder Vermikulargraphit aufweisen.

Mit dem erfindungsgemässen Verfahren wird erreicht, dass während der Magnesiumbehandlung der Gusseisenschmelze ohne grösseren Analysenaufwand innert kürzester Zeit eine Aussage über die metallographische Struktur des Gusseisens möglich wird und dass deshalb eventuell auftretende Fehlchargen in der industriellen Herstellung von Gusseisenformteilen früher als bisher erkannt werden.

Ein Ausführungsbeispiel der Erfindung wird anhand der Figur beschrieben.
Es zeigt:

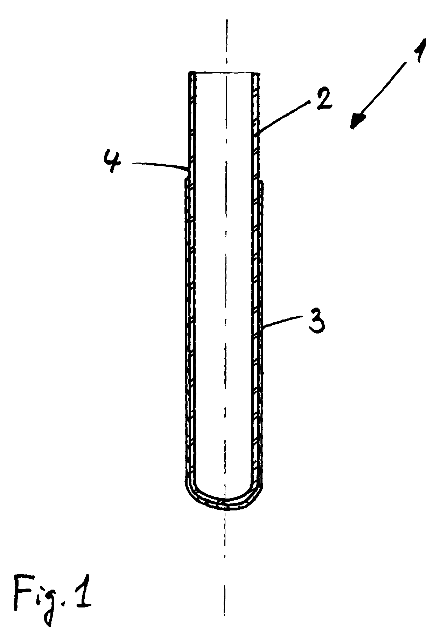

Figur 1 einen Schnitt durch einen erfindungsgemässen Sensor.

In Figur 1 ist ein Sensor 1 zur Bestimmung des Gehaltes an gelöstem Magnesium in einer Schmelze einer Gusseisenlegierung dargestellt. Der Sensor 1 aus Figur 1 besteht aus einem Träger 2, auf dem eine Mischung 3 von Metalloxiden und Schwefel aufgebracht wurde. Als Träger wird beispielsweise ein Schutzrohr 2 verwendet, das aus Quarzglas hergestellt wird. Solche Schutzrohre sind im Handel erhältlich und dienen unter anderem als Schutzrohr für Thermoelemente, die für die Temperaturmessung in Metallschmelzen verwendet werden. Anstelle des Rohres 2 kann auch eine andere Form des Trägers, beispielsweise ein zylindrischer Stab oder eine Platte gewählt werden. Wichtig ist, dass das Trägermaterial thermisch derart stabil ist, dass es die Temperatur der Gusseisenschmelze, die bis zu 1600 C° erreichen kann, für die Dauer der Messung aushält. Als Trägermaterial kann beispielsweise auch Bornitrid, Siliziumnitrid, Chromoxid oder Aluminiumoxid gewählt werden. Zumindest eine Aussenfläche 4 des Trägers 2 ist mattiert oder aufgerauht, damit die Mischung 3 gut an der Aussenfläche 4 haften bleibt.

Durch Eintauchen des Schutzrohres 2 in eine Lösung oder eine Suspension von Metalloxiden und Schwefel und durch anschliessendes Trocknen der an der Aussenfläche 4 anhaftende Schicht wird die Mischung 3 aufgebracht. Die Lösung oder die Suspension ist zusammengesetzt aus einer sogenannten im Handel erhältlichen Basisschlichte, die beispielsweise in Alkohol angerührt wird und aus Zusätzen, die in bestimmten Konzentrationen eine starke Abhängigkeit der Farbänderung in Funktion des Magnesiumgehaltes aufweisen. Eine gute Korrelation zwischen Farbänderung und Magnesiumgehalt zeigen beispielsweise eine Schlichte auf der Basis von Zirkonoxid mit Zusätzen von ca. 5 % Cr₂O₃, 5 bis 10 % Al₂O₃ und ca. 1 % Schwefel. Um einen grösseren Konzentrationsbereich von Magnesium in der Gusseisenschmelze erfassbar zu machen, können verschiedene Mischungen 3 von Metalloxiden eingesetzt werden. Als Metalloxiden kommen auch Zirkonoxid und Titanoxid in Anmerkung. Wenn die Gusseisenschmelze neben Magnesium weitere Metalle, wie beispielsweise Nickel, enthält, kann auch ohne Zusätze, d. h. mit einer reinen Schlichte auf der Basis von Zirkonoxid gearbeitet werden. Die Sensoren 1 sind, einmal getrocknet, chemisch stabil und während längerer Zeit haltbar, so dass eine Vielzahl von Sensoren 1 mit Identischen Eigenschaften auf einmal hergestellt und gelagert werden können. In der Beschichtung hat das Cr₂O₃ eine grüne Farbe. Wenn diese Beschichtung in eine Schmelze mit gelöstem Magnesium getaucht wird, bildet sich in der Beschichtung Magnesiumoxid, das eine weisse Farbe hat. Chromoxid wird durch die grössere Affinität des Sauerstoffs gegenüber Magnesium reduziert. Die grüne Farbe ändert sich und wird durch die weisse Farbe des Magnesiumoxids aufgehellt. Die Zugabe von Schwefel in der Beschichtung ist vorteilhaft für die Beschleunigung der Reaktion. In der Schmelze stehen die Anteile von Magnesiummetall, Magnesiumoxid und Magnesiumsulfid miteinander in Gleichgewicht. Ein Schwefelüberschuss in der Gusseisenschmelze verschiebt das Gleichgewicht in Richtung des freien Magnesiums. Der Schwefel in der Beschichtung bietet somit mehr Reaktionspartner für das Magnesium in der Schmelze an und unterstützt die Reaktion des Sensors 1 mit dem freien Magnesium.

Das Verfahren zur Bestimmung des Magnesiumgehaltes einer
Gusseisenschmelze mit einem oben beschriebenen Sensor 1 kann wie folgt beschrieben werden. Der Sensor 1 oder bei einem noch unbekannten Konzentrationsbereich die Sensoren werden höchstens 20 Sekunden in die Gusseisenschmelze eingetaucht. Hierbei ist darauf zu achten, dass die Schmelze eine möglichst konstante Temperatur hat und die Schmelze möglichst wenig bewegt wird. Der Sensor 1 wird senkrecht in die Schmelze eingetaucht und nach einer genau definierten Zeit wieder aus der Schmelze gezogen. Die Oberfläche der Schmelze soll möglichst frei von Schlacken sein, da sonst die Schlacke am Sensor anhaftet oder die Beschichtung des Sensors 1 beschädigt wird. Der Sensor 1 wird während der Eintauchzeit nicht bewegt. Anschliessend wird der Sensor etwa auf Raumtemperatur abgekühlt, damit der Sensor 1 weiter gehandhabt werden kann. Die Sensoraussenfläche 4 weist eine für den Magnesiumgehalt typische Farbänderung auf. Mit einer digitalen Videokamera wird ein möglichst grosser und einförmiger Bereich der Aussenfläche 4 unter standardisierten Belichtungsbedingungen, beispielsweise im Labor, fotografiert. Das digitale Bild kann mit einer Computersoftware für die digitale Farbbildbearbeitung ausgewertet werden. Hierbei können die Werte für die Hauptfarben Rot, Grün und Blau, sowie einen Wert für die Helligkeit bestimmt werden. Anhand von Eichkurven oder Referenzkurven, die im Computer gespeichert sind, kann auf dem Magnesiumgehalt geschlossen werden. Mit diesem Verfahren werden Gehalte von 50 bis 800 ppm Mg mit einer Genauigkeit von ± 20 ppm Mg bestimmt.

## Patentansprüche

1. Sensor (1) zur Bestimmung des Magnesiumgehaltes einer Gusseisenschmelze umfassend ein chemisch inerter Träger (2), auf dem eine Mischung (3) von Metalloxiden und Schwefel aufbringbar ist, wobei die Mischung (3) mit dem gelösten Magnesium der Gusseisenschmelze in Abhängigkeit des Gehaltes an gelöstem Magnesium unter Farbänderung irreversibel reagiert.

2. Sensor zur Bestimmung des Magnesiumgehaltes einer Gusseisenschmelze nach dem Anspruch 1, dadurch gekennzeichnet, dass als chemisch inerter Träger (2) ein Material gewählt wird, das bei der Temperatur der Gusseisenschmelze thermisch stabil ist.

3. Sensor zur Bestimmung des Magnesiumgehaltes einer Gusseisenschmelze nach dem Anspruch 1 oder 2, dadurch gekennzeichnet, dass als Träger (2) ein Material aus der Gruppe der Nitride von Silizium oder Bor oder aus der Gruppe der Oxiden von Aluminium, Silizium oder Chrom gewählt wird.

4. Sensor zur Bestimmung des Magnesiumgehaltes einer Gusseisenschmelze nach einem der Ansprüche 1 bis 3, dadurch gekennzeichnet, dass die mit dem Magnesium reagierenden Metalloxide aus der Gruppe der Oxide von Aluminium, Titan, Chrom, oder Zirkon gewählt wird.

5. Sensor zur Bestimmung des Magnesiumgehaltes einer Gusseisenschmelze nach einem der Ansprüche 1 bis 4, dadurch gekennzeichnet, dass zur Herstellung des Sensors (1) der Träger (2) in eine Lösung oder eine Suspension von Metalloxiden und Schwefel eingetaucht und anschliessend getrocknet wird, derart dass dabei eine Beschichtung des Trägers (2) mit der Mischung (3) erhalten wird.

6. Sensor zur Bestimmung des Magnesiumgehaltes einer Gusseisenschmelze nach einem der Ansprüche 1 bis 5, dadurch gekennzeichnet, dass die Mischung (3) von Metalloxiden und Schwefel aus einer zirkonoxidhaltigen Basisschlichte mit Zugaben von Aluminiumoxiden und/oder Chromoxiden zusammengesetzt ist.

7. Verfahren zur Bestimmung des Magnesiumgehaltes einer Gusseisenschmelze unter Verwendung des Sensors (1) nach einem der Ansprüche 1 bis 6, umfassend folgende Schritte:
- Eintauchen des Sensors in die Metallschmelze während 2 bis 20 Sekunden,
- Kühlen des Sensors auf Raumtemperatur,
- Beleuchten des Sensors mit einer Standardlichtquelle,
- Fotografieren des Sensors mit einem Farbfotoverfahren,
- Auswerten der Farbe und die Helligkeit des Sensors unter Verwendung einer Standardfarbskala und
- Vergleichen der Farb- und Helligkeitswerten mit einer Vergleichskurve.

8. Verfahren zur Bestimmung des Magnesiumgehaltes einer Gusseisenschmelze nach dem Anspruch 7, dadurch gekennzeichnet, dass die Verfahrensschritte Fotografieren, Auswerten und Vergleichen unter Verwendung einer digitalen Videokamera und einer damit gekoppelten Computersoftware für digitale Farbbildbearbeitung durchgeführt werden.
